# EUROPEAN PATENT APPLICATION

(11) **EP 4 756 009 A1**
(43) Date of publication of application: **10.06.2026**
(21) Application number: 24848988.2
(22) Date of filing: 22.07.2024
(51) Int. Cl.: C12M 1/34, G01N 33/15, G01N 33/50

(54) **INFORMATION PROCESSING DEVICE, OPERATION METHOD FOR INFORMATION PROCESSING DEVICE, AND OPERATION PROGRAM FOR INFORMATION PROCESSING DEVICE**

(30) Priority: 28.07.2023 JP 2023123812
(71) Applicant: FUJIFILM Corporation, Tokyo 106-8620 (JP)
(72) Inventor: MURAKAMI, Ryoichi, Ashigarakami-gun, Kanagawa 258-8577 (JP); HIKIDA, Yasushi, Ashigarakami-gun, Kanagawa 258-8577 (JP); TATESHITA, Masakazu, Ashigarakami-gun, Kanagawa 258-8577 (JP); SUGIYAMA, Satoshi, Ashigarakami-gun, Kanagawa 258-8577 (JP); HARA, Mika, Ashigarakami-gun, Kanagawa 258-8577 (JP)
(74) Representative: HGF
(86) International application number: PCT/JP2024/026195
(87) International publication number: WO 2025/028333

(57) **Abstract**

There is provided an information processing apparatus that predicts an evaluation result of an Ames mutagenicity test for a candidate substance for a product using a prediction model, the information processing apparatus including a processor, in which the processor is configured to: use, as the prediction model, a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation; acquire candidate substance information related to the candidate substance; input the candidate substance information or information based on the candidate substance information to the first prediction model and the second prediction model, and output the first prediction evaluation result and the second prediction evaluation result from the first prediction model and the second prediction model; and present prediction information corresponding to the first prediction evaluation result and the second prediction evaluation result to a user.

## Description

### BACKGROUND OF THE INVENTION

### 1. Field of the Invention

The technology of the present disclosure relates to an information processing apparatus, an operation method of an information processing apparatus, and an operation program of an information processing apparatus.

### 2. Description of the Related Art

In a drug discovery field and the like, an Ames mutagenicity test (also referred to as a reverse mutation test) is actively performed. The Ames mutagenicity test is a test for evaluating whether or not a candidate substance for a product such as a drug has mutagenicity. Mutagenicity is a property of causing irreversible mutations in a gene, and is one of the factors inducing carcinogenesis. In the Ames mutagenicity test, a candidate substance is added to bacteria such as Salmonella typhimurium, and whether or not the candidate substance has mutagenicity (positive) or whether or not the candidate substance does not have mutagenicity (negative) is evaluated based on a subsequent amount of proliferation of the bacteria.

As types of mutations of a gene, there are a base pair substitution mutation in which a part of a base sequence is changed, and a frameshift mutation in which a reading frame of a base sequence in units of three is shifted due to insertion or deletion of a base. In the Ames mutagenicity test, for example, it is recommended to use three strains TA100, TA1535, and WP2uvrA sensitive to the base pair substitution mutation and two strains TA98 and TA1537 sensitive to the frameshift mutation. In a case where any one of the five strains is positive, the candidate substance is rejected as having mutagenicity.

The Ames mutagenicity test has various problems such as a test period of one candidate substance being several weeks to several months, relatively high cost, a relatively large amount of candidate substance being required, and the test not being possible with a small amount. In addition, in some products, the number of candidate substances may be several thousand to tens of thousand, so it is unrealistic to actually perform the Ames mutagenicity test on all candidate substances. Therefore, in the related art, various technologies have been developed to predict an evaluation result of the Ames mutagenicity test using a machine learning model. For example, in M. J. Martinez, et al "Multitask Deep Neural Networks for Ames Mutagenicity Prediction" Journal of Chemical Information and Modeling 62 (24) September 2022. (hereinafter, referred to as Non-Patent Document 1), a technology of predicting an evaluation result of the Ames mutagenicity test using a multitask deep neural network model (hereinafter, simply referred to as a deep learning model) is described.

### SUMMARY OF THE INVENTION

However, the number of past Ames mutagenicity test data that can be used as training data of the deep learning model described in Non-Patent Document 1 is not so large, and is about several thousand in a case where data of all five strains are available. In addition, the deep learning model described in Non-Patent Document 1 is used in common for both the base pair substitution mutation and the frameshift mutation, which have completely different causes of occurrence, structural features, and the like. Therefore, the deep learning model described in Non-Patent Document 1 may have insufficient prediction accuracy.

One embodiment according to the technology of the present disclosure provides an information processing apparatus, an operation method of the information processing apparatus, and an operation program of the information processing apparatus, which can improve prediction accuracy of an evaluation result of an Ames mutagenicity test and identification accuracy of whether a type of a mutation of a gene is a base pair substitution mutation or a frameshift mutation.

There is provided an information processing apparatus according to the present disclosure that predicts an evaluation result of an Ames mutagenicity test for a candidate substance for a product using a prediction model, the information processing apparatus including a processor, in which the processor is configured to: use, as the prediction model, a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation; acquire candidate substance information related to the candidate substance; input the candidate substance information or information based on the candidate substance information to the first prediction model and the second prediction model, and output the first prediction evaluation result and the second prediction evaluation result from the first prediction model and the second prediction model; and present prediction information corresponding to the first prediction evaluation result and the second prediction evaluation result to a user.

It is preferable that the processor is configured to: present, in a case where both the first prediction evaluation result and the second prediction evaluation result indicate that the candidate substance does not have mutagenicity, a fact that the candidate substance does not have mutagenicity to the user as the prediction information; and present, in a case where at least one of the first prediction evaluation result or the second prediction evaluation result indicates that the candidate substance has mutagenicity, a fact that the candidate substance has mutagenicity to the user as the prediction information.

It is preferable that the processor is configured to present the first prediction evaluation result and the second prediction evaluation result themselves to the user as the prediction information.

It is preferable that the candidate substance information is information related to a chemical structure of the candidate substance.

It is preferable that the prediction model is constructed by any machine learning method of a support vector machine, linear separation, a gradient boosting tree, AdaBoost, a random forest, deep learning, or ensemble learning thereof.

It is preferable that the first prediction model is constructed by deep learning, and the second prediction model is constructed by performing transfer learning on the trained first prediction model.

It is preferable that the first prediction model and the second prediction model are constructed by different machine learning methods.

It is preferable that the first prediction model is constructed by deep learning, and the second prediction model is constructed by a machine learning method other than deep learning.

It is preferable that the first prediction model and the second prediction model are constructed by the same machine learning method while internal parameters for deriving the first prediction evaluation result and the second prediction evaluation result are different.

It is preferable that in a case where the prediction model is constructed by any machine learning method of a support vector machine, linear separation, a gradient boosting tree, AdaBoost, a random forest, or ensemble learning thereof, the processor is configured to: acquire feature amount information of the candidate substance; and input the feature amount information to the prediction model.

It is preferable that the feature amount information includes at least one of a feature amount related to a geometric shape of the candidate substance, a feature amount related to an electronic physical property of the candidate substance, a feature amount related to a physicochemical property of the candidate substance, or a feature amount related to a partial structure of the candidate substance.

It is preferable that first training data used for training the first prediction model and second training data used for training the second prediction model are at least partially different from each other.

It is preferable that first training data used for training the first prediction model and second training data used for training the second prediction model are prepared based on information on the first strain and the second strain.

There is provided an operation method of an information processing apparatus according to the present disclosure that predicts an evaluation result of an Ames mutagenicity test for a candidate substance for a product using a prediction model, the operation method including: using, as the prediction model, a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation; acquiring candidate substance information related to the candidate substance; inputting the candidate substance information or information based on the candidate substance information to the first prediction model and the second prediction model, and outputting the first prediction evaluation result and the second prediction evaluation result from the first prediction model and the second prediction model; and presenting prediction information corresponding to the first prediction evaluation result and the second prediction evaluation result to a user.

There is provided an operation program of an information processing apparatus according to the present disclosure that predicts an evaluation result of an Ames mutagenicity test for a candidate substance for a product using a prediction model, the operation program causing a computer to execute a process including: using, as the prediction model, a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation; acquiring candidate substance information related to the candidate substance; inputting the candidate substance information or information based on the candidate substance information to the first prediction model and the second prediction model, and outputting the first prediction evaluation result and the second prediction evaluation result from the first prediction model and the second prediction model; and presenting prediction information corresponding to the first prediction evaluation result and the second prediction evaluation result to a user.

According to the technology of the present disclosure, it is possible to provide an information processing apparatus, an operation method of the information processing apparatus, and an operation program of the information processing apparatus, which can improve prediction accuracy of an evaluation result of an Ames mutagenicity test.

### BRIEF DESCRIPTION OF THE DRAWINGS

FIG. 1 is a diagram showing an information processing server and a user terminal.
FIG. 2 is a block diagram showing a computer constituting the information processing server and the user terminal.
FIG. 3 is a block diagram showing a processing unit of a CPU of the information processing server.
FIG. 4 is a diagram showing feature amount information.
FIG. 5 is a diagram showing processing of a prediction unit that inputs candidate substance information to a first prediction model and causes the first prediction model to output a first prediction evaluation result.
FIG. 6 is a diagram showing processing of the prediction unit that inputs the feature amount information to a second prediction model and causes the second prediction model to output a second prediction evaluation result.
FIG. 7 is a diagram showing processing in a training phase of the first prediction model.
FIG. 8 is a diagram showing processing in a training phase of the second prediction model.
FIG. 9 is a diagram showing allocation of first training data and second training data.
FIG. 10 is a table showing the first prediction evaluation result, the second prediction evaluation result, and an overall prediction evaluation result.
FIG. 11 is a diagram showing prediction information.
FIG. 12 is a block diagram showing a processing unit of a CPU of the user terminal.
FIG. 13 is a diagram showing an information input screen.
FIG. 14 is a diagram showing a prediction evaluation result display screen.
FIG. 15 is a flowchart showing a procedure of processing of the information processing server.
FIG. 16 is a diagram showing an aspect in which the second prediction model is constructed by performing transfer learning on a trained first prediction model.
FIG. 17 is a diagram showing another method of prediction using the first prediction model and the second prediction model.
FIG. 18 is a diagram showing another example of the allocation of the first training data and the second training data.
FIG. 19 is a diagram showing both the first prediction model and the second prediction model constructed by a support vector machine.
FIG. 20 is a diagram showing a boundary line in a feature amount space of the first prediction model shown in FIG. 19.
FIG. 21 is a diagram showing a boundary line in a feature amount space of the second prediction model shown in FIG. 19.

### DESCRIPTION OF THE PREFERRED EMBODIMENTS

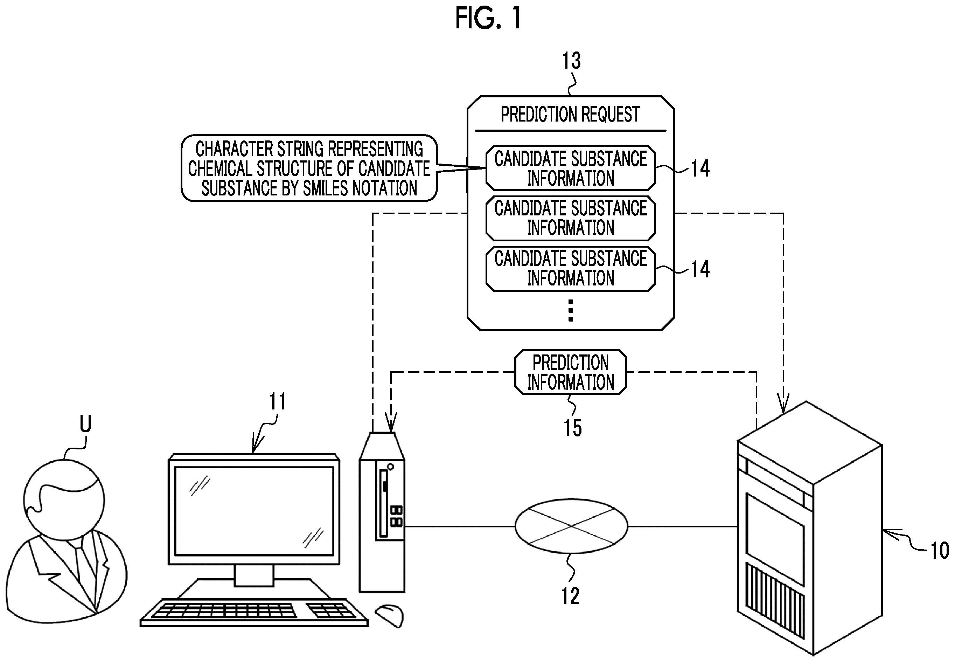

As shown in FIG. 1 as an example, an information processing server 10 is connected to a user terminal 11 via a network 12. The information processing server 10 is an example of an "information processing apparatus" according to the technology of the present disclosure. The user terminal 11 is installed in, for example, a pharmaceutical company that develops a drug as a product, or an institution that receives a development business of a drug from the pharmaceutical company, that is, a contract research organization (CRO). The user terminal 11 is operated by a user U who is involved in the development of a drug in the pharmaceutical company or the CRO. The network 12 is, for example, a wide area network (WAN) such as the Internet or a public communication network. In FIG. 1, only one user terminal 11 is connected to the information processing server 10, but in reality, a plurality of user terminals 11 of a plurality of pharmaceutical companies or CROs are connected to the information processing server 10.

The user terminal 11 transmits a prediction request 13 to the information processing server 10. The prediction request 13 is a request for the information processing server 10 to predict an evaluation result of an Ames mutagenicity test for a candidate substance of a drug. The prediction request 13 includes candidate substance information 14 related to the candidate substance. In a case where there are a plurality of candidate substances for which the evaluation result of the Ames mutagenicity test is desired to be predicted, the prediction request 13 includes a plurality of pieces of candidate substance information 14 corresponding to a plurality of candidate substances as shown in the drawing. The candidate substance information 14 is information related to a chemical structure of the candidate substance. More specifically, the candidate substance information 14 is a character string representing the chemical structure of the candidate substance by a simplified molecular input line entry system (SMILES) notation. Although not shown, the prediction request 13 also includes a terminal identification data (ID) or the like for uniquely identifying the user terminal 11 which is a transmission source of the prediction request 13.

In a case where the prediction request 13 is received, the information processing server 10 predicts the evaluation result of the Ames mutagenicity test for the candidate substance to derive prediction information 15. The information processing server 10 delivers the prediction information 15 to the user terminal 11 that is the transmission source of the prediction request 13. In a case where the prediction information 15 is received, the user terminal 11 provides the prediction information 15 for browsing by the user U.

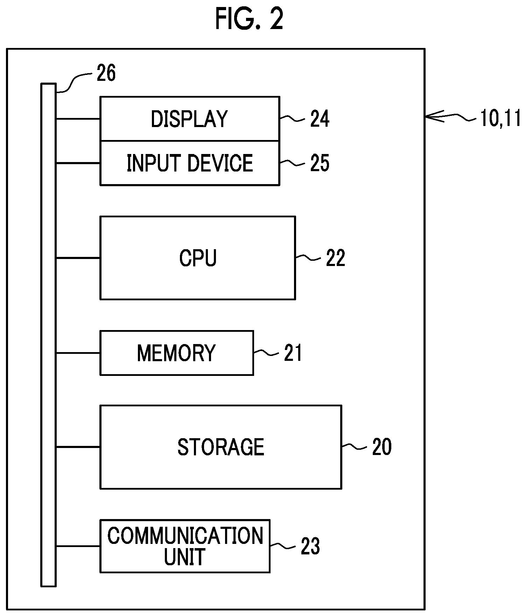

As shown in FIG. 2 as an example, computers constituting the information processing server 10 and the user terminal 11 basically have the same configuration, and comprise a storage 20, a memory 21, a central processing unit (CPU) 22, a communication unit 23, a display 24, and an input device 25. These are connected to each other via a bus line 26.

The storage 20 is a hard disk drive that is built in the computers constituting the information processing server 10 and the user terminal 11 or connected thereto through a cable or a network. Alternatively, the storage 20 is a disk array, with a plurality of hard disk drives connected in parallel. The storage 20 stores a control program such as an operating system, various application programs (hereinafter, referred to as an application program (AP)), various types of data associated with these programs, and the like. A solid state drive may be used instead of the hard disk drive.

The memory 21 is a work memory for the CPU 22 to execute processing. The CPU 22 loads the program stored in the storage 20 to the memory 21, and executes processing in accordance with the program. Thus, the CPU 22 collectively controls the respective units of the computer. The CPU 22 is an example of a "processor" according to the technology of the present disclosure. Note that the memory 21 may be incorporated into the CPU 22.

The communication unit 23 is a network interface that performs control of transmitting various types of information via a network 12 and the like. The display 24 displays various screens. The various screens comprise an operation function by a graphical user interface (GUI). The computers constituting the information processing server 10 and the user terminal 11 receive input of an operation instruction from the input device 25 through various screens. The input device 25 is a keyboard, a mouse, a touch panel, a microphone for voice input, and the like.

Further, in the following description, the subscript "A" is attached to the reference numerals indicating each unit (the storage 20 and the CPU 22) of the computer constituting the information processing server 10, and the subscript "B" is attached to the reference numerals indicating each unit (the storage 20, the CPU 22, the display 24, and the input device 25) of the computer constituting the user terminal 11 to distinguish the units.

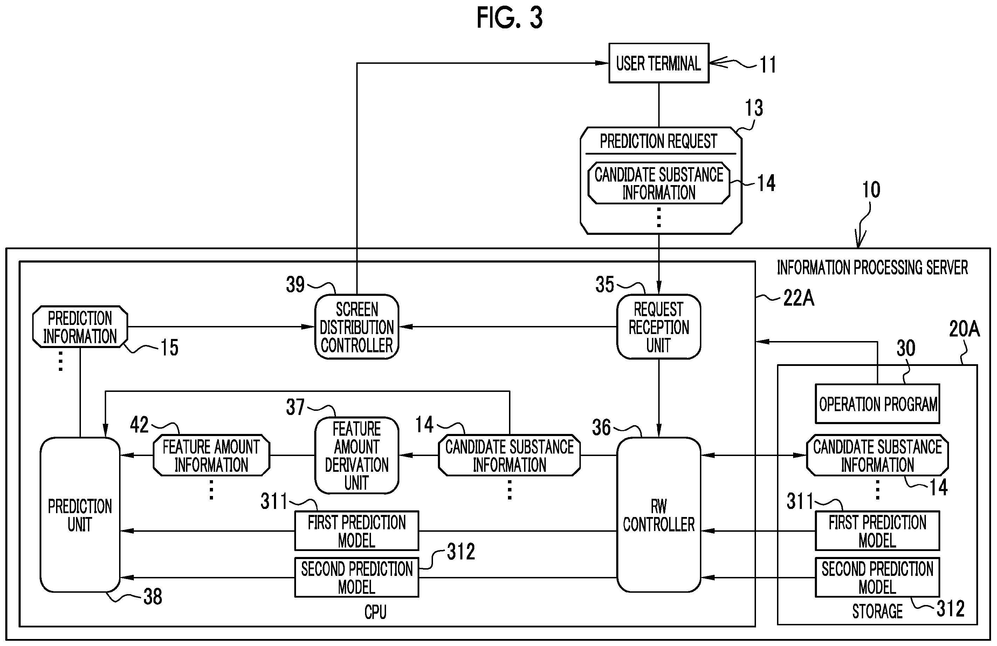

For example, as shown in FIG. 3, an operation program 30 is stored in a storage 20A of the information processing server 10. The operation program 30 is an AP for causing the computer to function as the information processing server 10. That is, the operation program 30 is an example of "an operation program of the information processing apparatus" according to the technology of the present disclosure. The storage 20 also stores a first prediction model 311, a second prediction model 312, and the like. The first prediction model 311 and the second prediction model 312 are examples of a "prediction model" according to the technology of the present disclosure. In the following description, in a case where there is no need to particularly distinguish between the first prediction model 311 and the second prediction model 312, the first prediction model 311 and the second prediction model 312 are collectively referred to as a prediction model 31.

In a case where the operation program 30 is started, the CPU 22A of the computer constituting the information processing server 10 functions as a request reception unit 35, a read and write (hereinafter, abbreviated to RW) control unit 36, a feature amount derivation unit 37, a prediction unit 38, and a screen delivery control unit 39 in cooperation with the memory 21 and the like.

The request reception unit 35 receives various requests from the user terminal 11, including the prediction request 13. As described above, the prediction request 13 includes the candidate substance information 14. Therefore, the request reception unit 35 acquires the candidate substance information 14 by receiving the prediction request 13. In a case where the prediction request 13 is received, the request reception unit 35 outputs the candidate substance information 14 included in the prediction request 13 to the RW control unit 36. In addition, the request reception unit 35 outputs the terminal ID of the user terminal 11 included in the prediction request 13 to the screen delivery control unit 39.

The RW control unit 36 controls storage of various types of data in the storage 20A and readout of various types of data from the storage 20A. In particular, the RW control unit 36 controls storage of the candidate substance information 14 in the storage 20A and reading out of the candidate substance information 14 from the storage 20A. The RW control unit 36 outputs the read candidate substance information 14 to the feature amount derivation unit 37 and the prediction unit 38. In addition, the RW control unit 36 reads out the first prediction model 311 and the second prediction model 312 from the storage 20A, and outputs the read first prediction model 311 and second prediction model 312 to the prediction unit 38.

The feature amount derivation unit 37 derives feature amount information 42 of the candidate substance from the candidate substance information 14. More specifically, the feature amount derivation unit 37 derives one piece of feature amount information 42 from the candidate substance information 14 of one candidate substance. Therefore, the feature amount derivation unit 37 derives the same number of pieces of feature amount information 42 as the candidate substance information 14 included in the prediction request 13. The feature amount derivation unit 37 derives the feature amount information 42 by using, for example, a machine learning model that outputs the feature amount information 42 in a case where the candidate substance information 14 is input. The feature amount derivation unit 37 outputs the feature amount information 42 to the prediction unit 38.

The prediction unit 38 causes the first prediction model 311 and the second prediction model 312 to predict the evaluation result of the Ames mutagenicity test for the candidate substance based on the candidate substance information 14 and the feature amount information 42. The prediction unit 38 generates the prediction information 15 and outputs the prediction information 15 to the screen delivery control unit 39.

The screen delivery control unit 39 performs control of delivering various screens to the user terminal 11. Specifically, the screen delivery control unit 39 delivers output of the various screens to the user terminal 11 that is a transmitter of the various requests, in the form of screen data for web delivery created using a markup language such as extensible markup language (XML). In this case, the screen delivery control unit 39 specifies the user terminal 11 that is the transmission source of various requests based on the terminal ID from the request reception unit 35. Note that, instead of XML, another data description language, such as JavaScript (registered trademark) Object Notation (JSON), may be used.

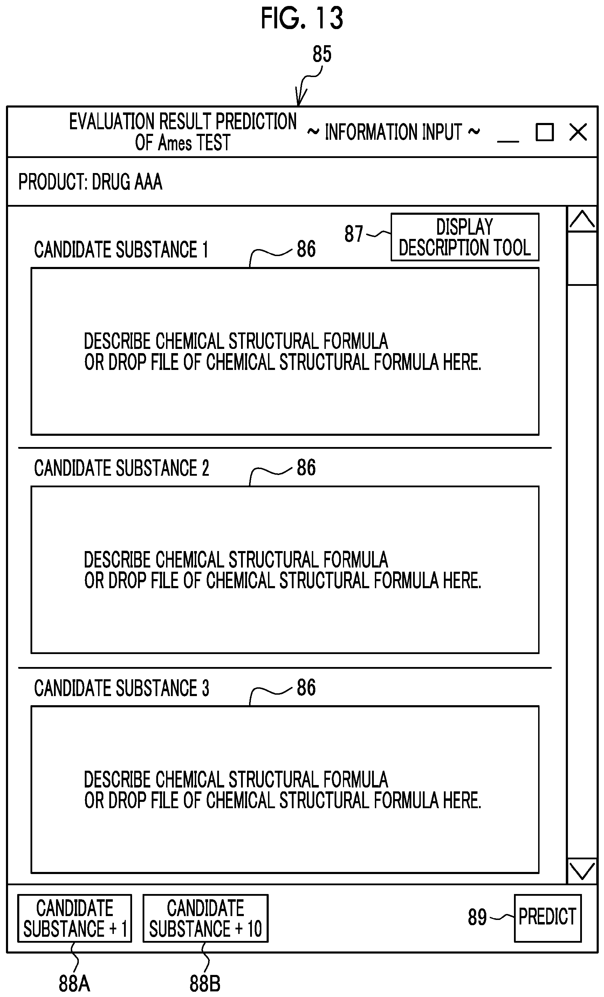

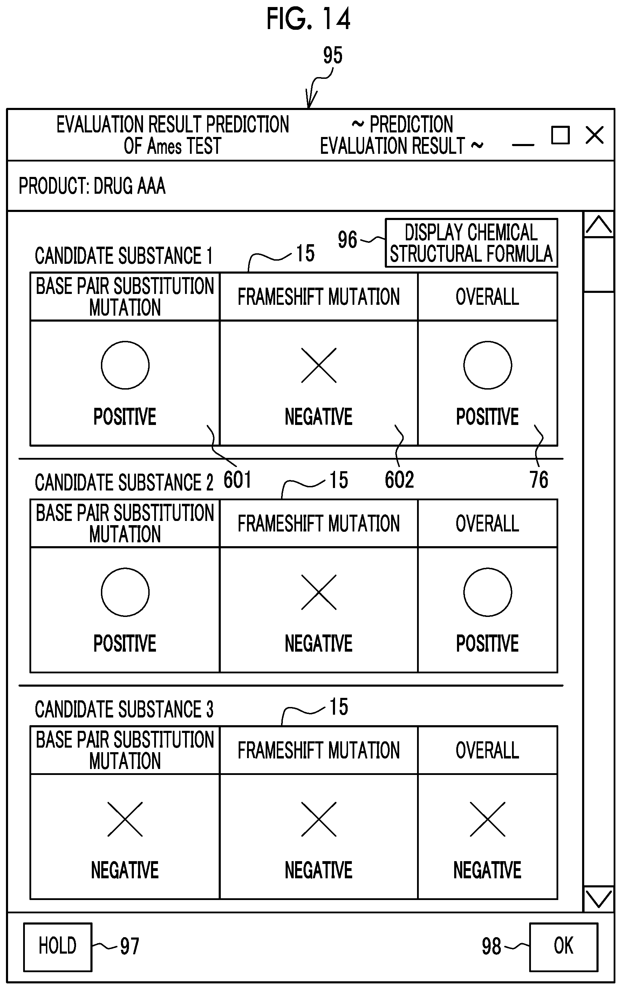

The various screens include an information input screen 85 (see FIG. 13) for inputting the candidate substance information 14, a prediction evaluation result display screen 95 (see FIG. 14) for displaying the prediction information 15, and the like. In addition to each of the processing units 35 to 39, an instruction reception unit that receives various operation instructions from the input device 25, or the like is also constructed in the CPU 22A.

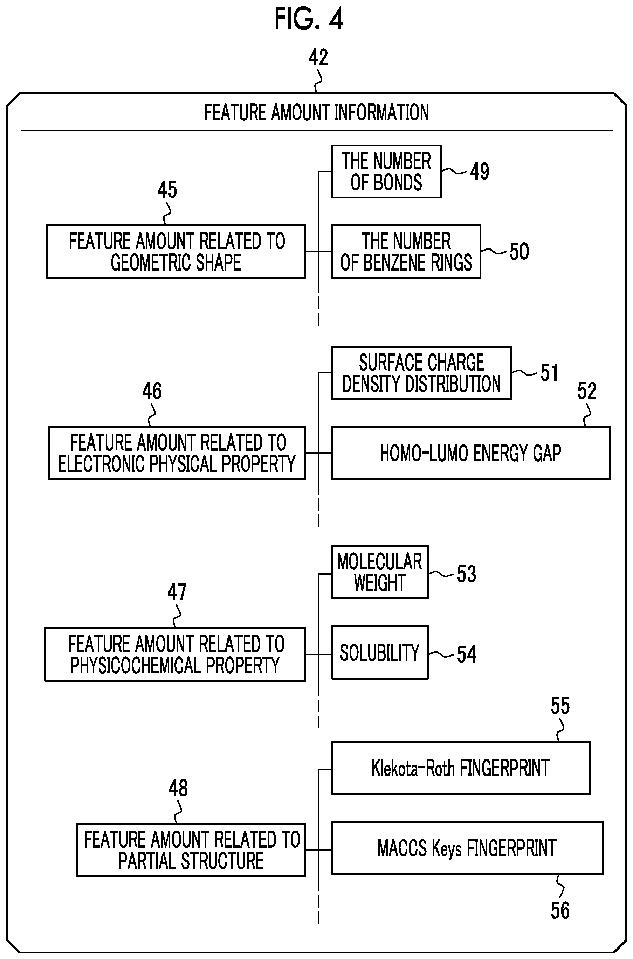

As shown in FIG. 4 as an example, the feature amount information 42 includes a feature amount 45 related to a geometric shape of the candidate substance, a feature amount 46 related to an electronic physical property of the candidate substance, a feature amount 47 related to a physicochemical property of the candidate substance, and a feature amount 48 related to a partial structure of the candidate substance. The feature amount 45 related to the geometric shape includes the number 49 of bonds of the candidate substance, the number 50 of benzene rings of the candidate substance, and the like. The feature amount 46 related to the electronic physical property includes a surface charge density distribution 51 of the candidate substance, a highest occupied molecular orbital (HOMO)-lowest unoccupied molecular orbital (LUMO) energy gap 52 of the candidate substance, and the like.

The feature amount 47 related to the physicochemical property includes a molecular weight 53 of the candidate substance, a solubility 54 in water indicating hydrophilicity and hydrophobicity of the candidate substance, and the like. The feature amount 48 related to the partial structure includes a Klekota-Roth fingerprint 55 of the candidate substance derived by a partial structure extraction algorithm, a MACCS Keys fingerprint 56 of the candidate substance, and the like. The feature amount 48 related to the partial structure may include a topological fingerprint, a Morgan fingerprint, a MinHash fingerprint, an Avalon fingerprint, an atom pair fingerprint, a topological dihedral angle fingerprint, a PubChem fingerprint, and the like.

The various feature amounts of the feature amount information 42 are selected by the developer of the operation program 30 as being useful for predicting whether or not the candidate substance has mutagenicity related to the frameshift mutation. The number of feature amounts included in the feature amount information 42 is preferably 200 or more and more preferably 1,000 or more. Therefore, the feature amount information 42 can be described as data of a multi-dimensional feature amount having several hundred to several thousand dimensions.

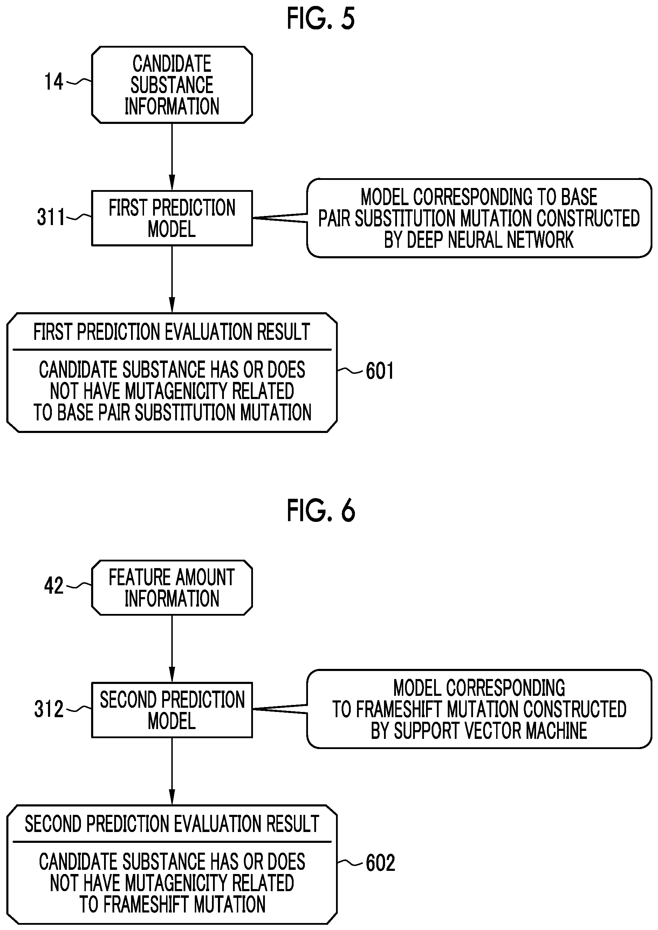

As shown in FIG. 5 as an example, the prediction unit 38 inputs the candidate substance information 14 to the first prediction model 311 and causes the first prediction model 311 to output a first prediction evaluation result 601. The first prediction evaluation result 601 indicates whether or not the candidate substance has mutagenicity related to the base pair substitution mutation in a case where the candidate substance is added to the first strain having sensitivity to the base pair substitution mutation in which a part of a base sequence is changed.

In addition, as shown in FIG. 6 as an example, the prediction unit 38 inputs the feature amount information 42 derived from the candidate substance information 14 to the second prediction model 312 and causes the second prediction model 312 to output a second prediction evaluation result 602. The feature amount information 42 is an example of "information based on candidate substance information" according to the technology of the present disclosure. The second prediction evaluation result 602 indicates whether or not the candidate substance has mutagenicity related to the frameshift mutation in a case where the candidate substance is added to the second strain having sensitivity to the frameshift mutation in which a reading frame of a base sequence is shifted.

Here, as shown in FIG. 5, the first prediction model 311 is a deep neural network, that is, a machine learning model corresponding to the base pair substitution mutation, which is constructed by deep learning. On the other hand, as shown in FIG. 6, the second prediction model 312 is a machine learning model corresponding to the frameshift mutation, which is constructed by a support vector machine. As described above, the first prediction model 311 and the second prediction model 312 are constructed by different machine learning methods. In addition, the first prediction model 311 is constructed by deep learning, and the second prediction model 312 is constructed by a machine learning method (here, a support vector machine) other than deep learning. The first prediction model 311 and the second prediction model 312 may be constructed by any machine learning method of a support vector machine, linear separation, a gradient boosting tree, AdaBoost, a random forest, deep learning, or ensemble learning thereof.

As is clear from the above description, the first prediction model 311 and the second prediction model 312 are not models that are experimentally constructed by a method using only nucleic acid and a compound, such as an isothermal titration calorimeter or ultraviolet-visible spectrophotometry, which does not use a strain. In addition, the first prediction model 311 and the second prediction model 312 are not models that are constructed only by simulation such as docking simulation or quantum chemical calculation.

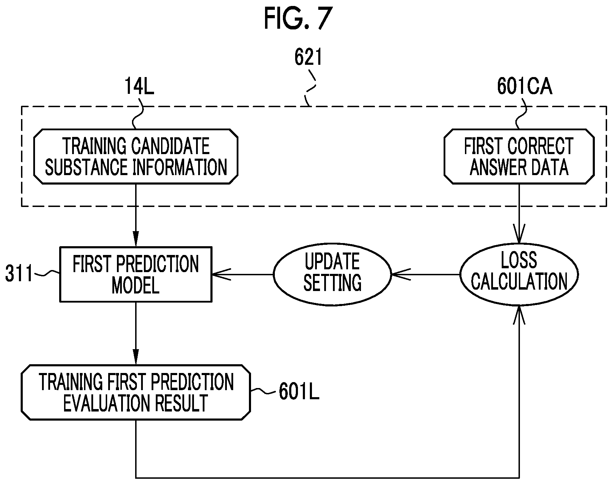

As shown in FIG. 7 as an example, the first prediction model 311 is trained by first training data 621. The first training data 621 is a set of training candidate substance information 14L and first correct answer data 601CA. The training candidate substance information 14L is candidate substance information 14 of a candidate substance for which the Ames mutagenicity test has been actually performed in the past. The first correct answer data 601CA is a result of evaluating whether or not the candidate substance of a provider of the training candidate substance information 14L has mutagenicity in the Ames mutagenicity test that has been actually performed in the past.

The training candidate substance information 14L is input to the first prediction model 311. As a result, a training first prediction evaluation result 601L is output from the first prediction model 311. The training first prediction evaluation result 601L is compared with the first correct answer data 601CA, and a loss calculation of the first prediction model 311 using a loss function is performed based on the comparison result. Then, an update setting of an internal parameter such as a coefficient of a filter of the first prediction model 311 is performed according to a result of the loss calculation, and the first prediction model 311 is updated according to the update setting.

The series of processing of inputting the training candidate substance information 14L to the first prediction model 311, outputting the training first prediction evaluation result 601L from the first prediction model 311, performing the loss calculation, performing the update setting, and updating the first prediction model 311 is repeatedly performed while the first training data 621 is changed. Then, in a case where the prediction accuracy of the training first prediction evaluation result 601L with respect to the first correct answer data 601CA reaches a level set in advance, the repetition of the series of processing is ended. In this way, the first prediction model 311 in which the prediction accuracy reaches the level set in advance is stored in the storage 20A. Regardless of the prediction accuracy, the training may be ended in a case where the series of types of processing have been repeated a predetermined number of times. In addition, the training of the first prediction model 311 may be continued even after being stored in the storage 20A.

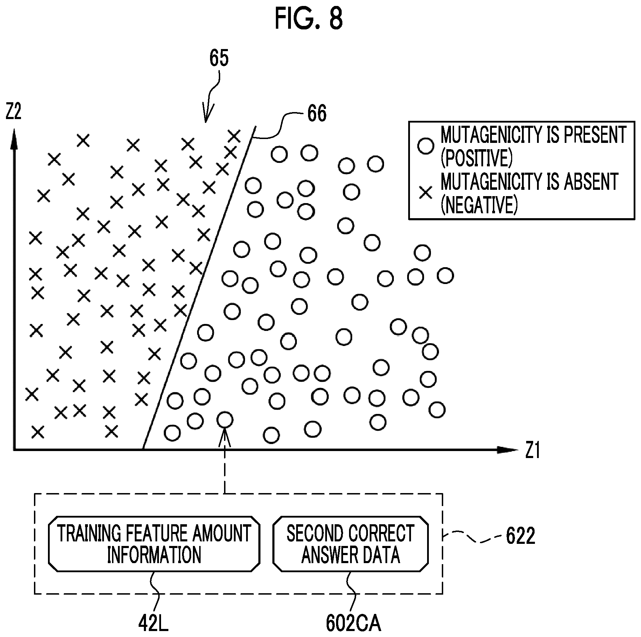

As shown in FIG. 8 as an example, the second prediction model 312 is generated based on second training data 622. The second training data 622 is a set of training feature amount information 42L and second correct answer data 602CA. The training feature amount information 42L is feature amount information 42 of a candidate substance for which the Ames mutagenicity test has been actually performed in the past. The second correct answer data 602CA is a result of evaluating whether or not the candidate substance of a provider of the training feature amount information 42L has mutagenicity in the Ames mutagenicity test that has been actually performed in the past.

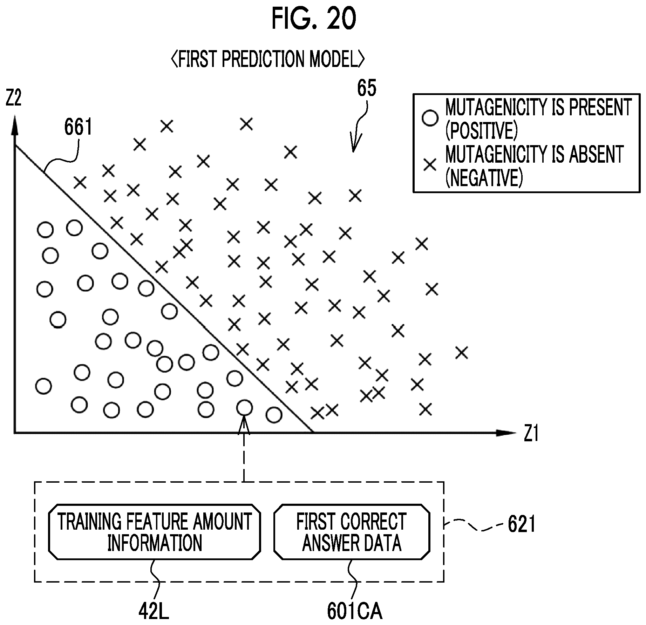

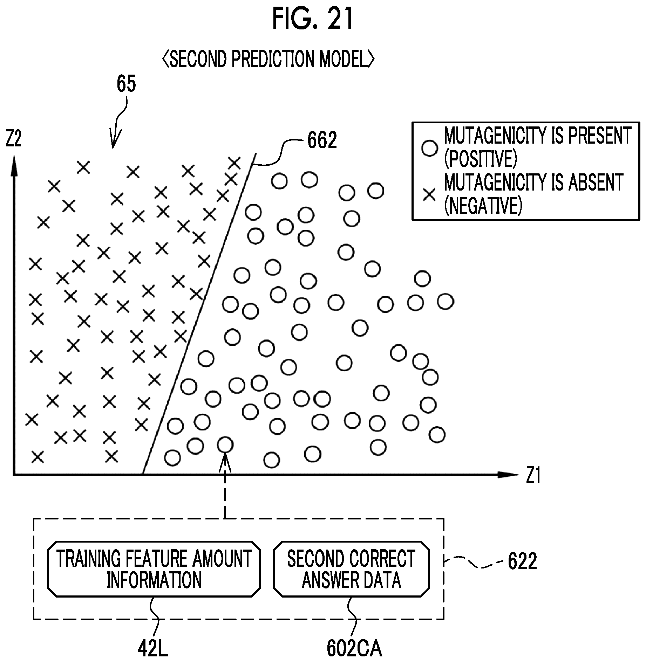

There are a plurality of pieces of the second training data 622, and there are pieces of the second training data 622 indicating that the second correct answer data 602CA has mutagenicity (positive) in the candidate substance and pieces of the second training data 622 indicating that the second correct answer data 602CA does not have mutagenicity (negative) in the candidate substance. In a graph in which the plurality of pieces of the second training data 622 are plotted in a feature amount space 65 represented by a plurality of feature amounts constituting the training feature amount information 42L, a boundary line 66 that can classify the second training data 622 indicating that the second correct answer data 602CA has mutagenicity in the candidate substance and the second training data 622 indicating that the second correct answer data 602CA does not have mutagenicity in the candidate substance is determined. In this case, an optimization problem of reducing misclassification while maximizing a margin, which is a distance from the boundary line 66 to the support vector, is solved for the second training data 622 close to the boundary line 66. In this way, the second prediction model 312 is generated by determining the boundary line 66. The generated second prediction model 312 is stored in the storage 20A. The training of the second prediction model 312 may be continued even after being stored in the storage 20A. In addition, in FIG. 8, for convenience of description, the dimension of the feature amount space 65 is set to two dimensions having a Z1 axis and a Z2 axis, but the actual dimension of the feature amount space 65 is several hundred to several thousand as described above. In FIGS. 20 and 21 as well, for convenience of description, the dimension of the feature amount space 65 is represented by two dimensions.

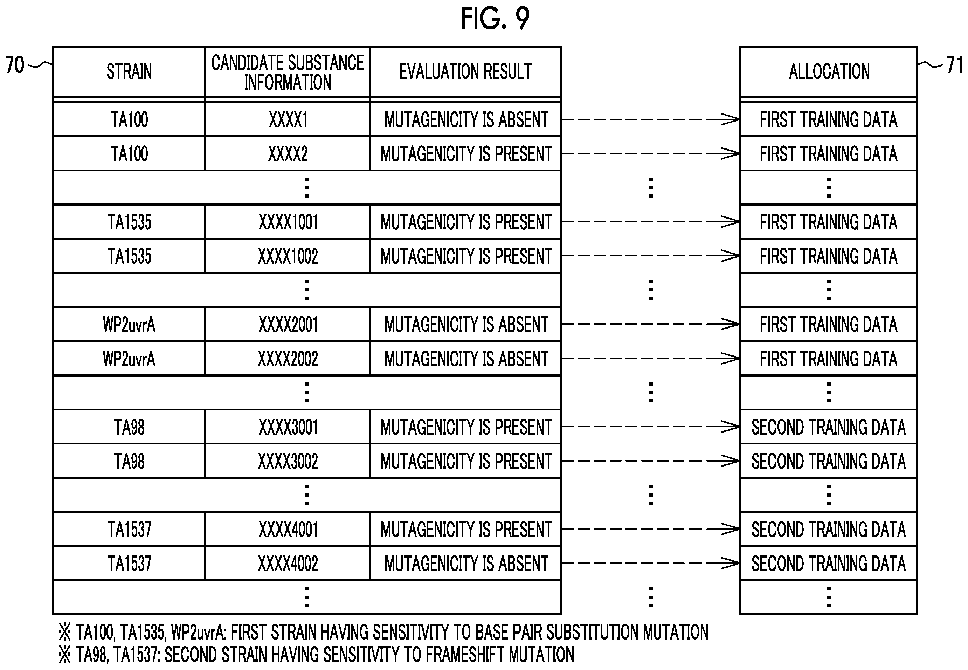

As shown in FIG. 9 as an example, in information 70 on the Ames mutagenicity test that has been actually performed in the past, which is the source of the first training data 621 and the second training data 622, information on the strain is registered together with the candidate substance information 14 and an evaluation result of whether or not the candidate substance has mutagenicity. As described above, the strain includes a first strain having sensitivity to the base pair substitution mutation and a second strain having sensitivity to the frameshift mutation. In the present example, the first strain is three types of TA100, TA1535, and WP2uvrA, and the second strain is two types of TA98 and TA1537. WP2uvrA/pKM101 may be used instead of WP2uvrA. Similarly, TA98NR may be used instead of TA98. In addition, the first strain may include TA102. Furthermore, the second strain may include TA97 or TA97a or TA1538.

As shown in Table 71, among the Ames mutagenicity tests that have been actually performed in the past, those in which the first strain is registered are allocated to the first training data 621, and those in which the second strain is registered are allocated to the second training data 622. That is, the first training data 621 and the second training data 622 are prepared based on the information on the first strain and the second strain. Therefore, the first training data 621 and the second training data 622 are different from each other.

The candidate substance information 14 of the Ames mutagenicity test allocated to the first training data 621 is training candidate substance information 14L of the first training data 621, and the evaluation result is the first correct answer data 601CA. In addition, the feature amount information 42 derived from the candidate substance information 14 of the Ames mutagenicity test allocated to the second training data 622 is training feature amount information 42L of the second training data 622, and the evaluation result is the second correct answer data 602CA. The information 70 on the Ames mutagenicity test that has been actually performed in the past may be generally widely published public information or information independently accumulated in the pharmaceutical company or the CRO. In addition, the information 70 may be composed of both the public information and the information independently accumulated in the pharmaceutical company or the CRO.

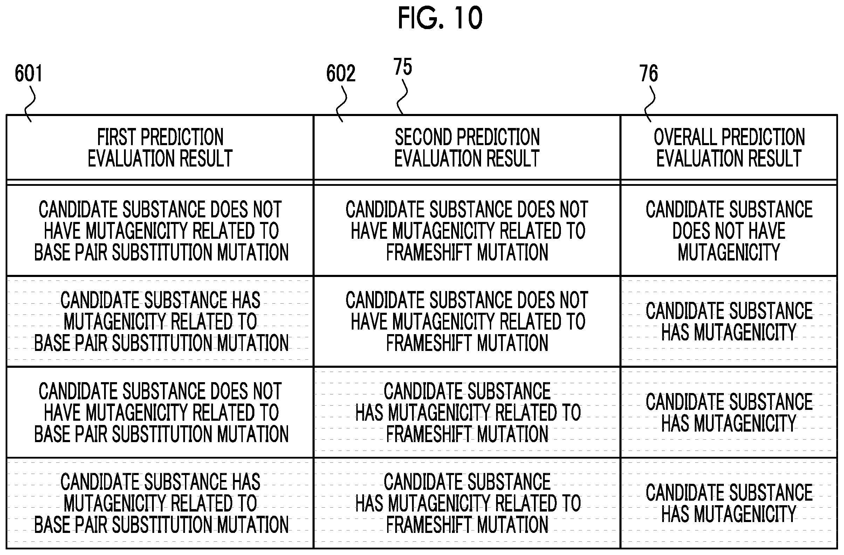

As shown in the uppermost column of Table 75 in FIG. 10 as an example, in a case where both the first prediction evaluation result 601 and the second prediction evaluation result 602 indicate that the candidate substance does not have mutagenicity, the prediction unit 38 outputs an overall prediction evaluation result 76 indicating that the candidate substance does not have mutagenicity. On the other hand, as shown in columns other than the uppermost column of Table 75, in a case where at least one of the first prediction evaluation result 601 or the second prediction evaluation result 602 indicates that the candidate substance has mutagenicity, the prediction unit 38 outputs the overall prediction evaluation result 76 indicating that the candidate substance has mutagenicity.

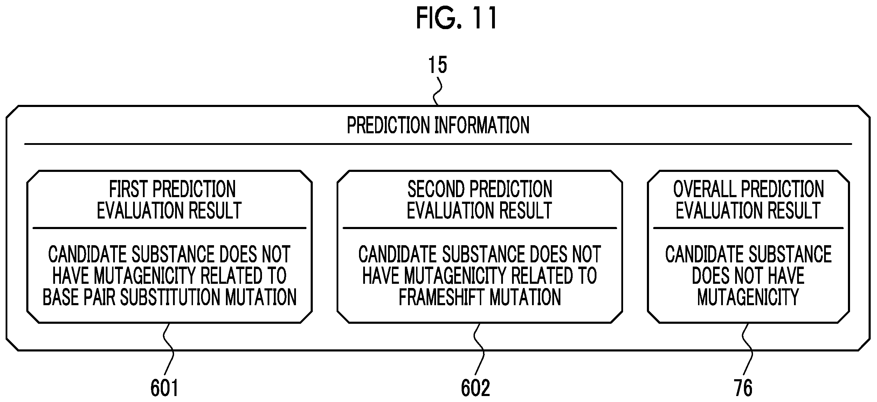

As shown in FIG. 11 as an example, the prediction unit 38 outputs the first prediction evaluation result 601, the second prediction evaluation result 602, and the overall prediction evaluation result 76 as the prediction information 15. FIG. 11 shows a case in which both the first prediction evaluation result 601 and the second prediction evaluation result 602 indicate that the candidate substance does not have mutagenicity.

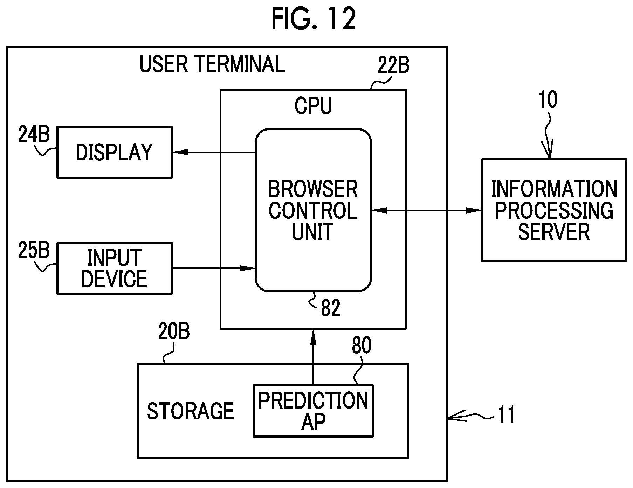

As shown in FIG. 12 as an example, a prediction AP 80 is stored in the storage 20B of the user terminal 11. The prediction AP 80 is installed in the user terminal 11 by the user U. The prediction AP 80 is an AP for predicting the evaluation result of the Ames mutagenicity test. In a case where the prediction AP 80 is activated, a CPU 22B of the user terminal 11 functions as a browser control unit 82 in cooperation with the memory 21 and the like. The browser control unit 82 controls an operation of a dedicated web browser of the prediction AP 80.

The browser control unit 82 reproduces various screens based on various types of screen data from the information processing server 10 and displays the reproduced various screens on the display 24B. Additionally, the browser control unit 82 receives various operation instructions input by the user U from the input device 25B through various screens. The browser control unit 82 transmits various requests corresponding to the operation instructions including the prediction request 13 to the information processing server 10.

In a case where the prediction AP 80 is activated, the information input screen 85 shown in FIG. 13 as an example is displayed on the display 24B under the control of the browser control unit 82. The information input screen 85 is provided with input boxes 86 for the candidate substance information 14 of a plurality of candidate substances. In the input box 86, the chemical structural formula of the candidate substance can be described by using a description tool that appears by selecting a description tool display button 87, or a file of the chemical structural formula of the candidate substance can be dropped. The input box 86 can be added by selecting addition buttons 88A and 88B at the bottom. The addition button 88A is a button for adding one input box 86, and the addition button 88B is a button for adding 10 input boxes 86.

The user U inputs a desired chemical structural formula of the candidate substance into the input box 86, and then selects a prediction button 89. In a case where the prediction button 89 is selected, the browser control unit 82 generates the prediction request 13 including the candidate substance information 14 corresponding to the chemical structural formula input to the input box 86, and transmits the generated prediction request 13 to the information processing server 10.

In addition, in a case where the prediction of the evaluation result of the Ames mutagenicity test is performed in the information processing server 10, the prediction evaluation result display screen 95 shown in FIG. 14 as an example is displayed on the display 24B under the control of the browser control unit 82. The prediction information 15 of each candidate substance is displayed in a list on the prediction evaluation result display screen 95. As described above, the prediction information 15 is presented to the user U in a form of delivery of screen data.

A chemical structural formula display button 96 is provided at the upper part of the prediction evaluation result display screen 95. In a case where the chemical structural formula display button 96 is selected, a list screen of the chemical structural formula of the candidate substance is displayed. In addition, a save button 97 and an OK button 98 are provided at the lower part of the prediction evaluation result display screen 95. In a case where the save button 97 is selected, the candidate substance information 14 and the prediction information 15 are stored in association with each other in the storage 20B of the user terminal 11. In a case where the OK button 98 is selected, the display of the prediction evaluation result display screen 95 is erased.

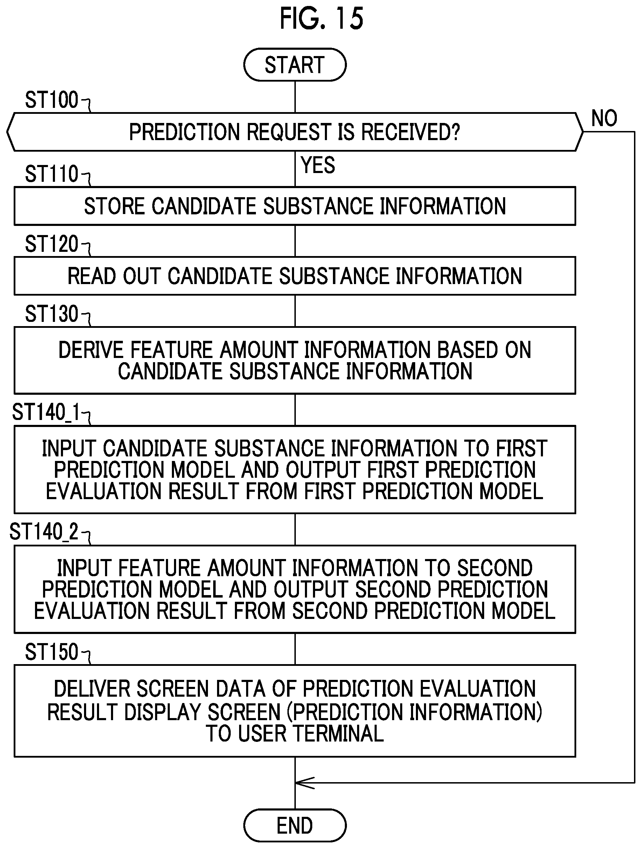

Next, an operation of the configuration described above will be described with reference to the flowchart shown in FIG. 15 as an example. In a case where the operation program 30 is activated in the information processing server 10, as shown in FIG. 3, the CPU 22A of the information processing server 10 functions as the request reception unit 35, the RW control unit 36, the feature amount derivation unit 37, the prediction unit 38, and the screen delivery control unit 39. In addition, in a case where the prediction AP 80 is activated in the user terminal 11, as shown in FIG. 12, the CPU 22B of the user terminal 11 functions as the browser control unit 82.

The information input screen 85 shown in FIG. 13 is displayed on the display 24B of the user terminal 11 under the control of the browser control unit 82. In a case where the user U inputs the chemical structural formula of the desired candidate substance into the input box 86 and selects the prediction button 89 on the information input screen 85, the prediction request 13 is transmitted from the browser control unit 82 to the information processing server 10. As shown in FIG. 1, the prediction request 13 includes the candidate substance information 14 that is a character string representing the chemical structure of the candidate substance by the SMILES notation, the terminal ID of the user terminal 11, and the like.

In the information processing server 10, the request reception unit 35 receives the prediction request 13 (YES in step ST100). The candidate substance information 14 included in the prediction request 13 is output to the RW control unit 36 from the request reception unit 35 and is stored in the storage 20A under control of the RW control unit 36 (step ST110). In addition, the terminal ID of the user terminal 11 included in the prediction request 13 is output from the request reception unit 35 to the screen delivery control unit 39.

The candidate substance information 14 is read out from the storage 20A by the RW control unit 36 (step ST120). The candidate substance information 14 is output from the RW control unit 36 to the feature amount derivation unit 37 and the prediction unit 38.

In the feature amount derivation unit 37, the feature amount information 42 is derived from the candidate substance information 14 (step ST130). As shown in FIG. 4, the feature amount information 42 includes the feature amount 45 related to the geometric shape of the candidate substance, the feature amount 46 related to the electronic physical property of the candidate substance, the feature amount 47 related to the physicochemical property of the candidate substance, and the feature amount 48 related to the partial structure of the candidate substance. The feature amount information 42 is output from the feature amount derivation unit 37 to the prediction unit 38.

In the prediction unit 38, as shown in FIG. 5, the candidate substance information 14 is input to the first prediction model 311. As a result, the first prediction evaluation result 601 is output from the first prediction model 311 (step ST140_1). In addition, in parallel with this, as shown in FIG. 6, the feature amount information 42 is input to the second prediction model 312 in the prediction unit 38. As a result, the second prediction evaluation result 602 is output from the second prediction model 312 (step ST140_2). As shown in FIG. 11, the prediction information 15 including the first prediction evaluation result 601, the second prediction evaluation result 602, and the overall prediction evaluation result 76 is generated. The prediction information 15 is output from the prediction unit 38 to the screen delivery control unit 39.

The screen delivery control unit 39 generates screen data of the prediction evaluation result display screen 95 shown in FIG. 14 based on the prediction information 15. The screen data of the prediction evaluation result display screen 95 is delivered to the user terminal 11 that is the transmission source of the prediction request 13 under the control of the screen delivery control unit 39 (step ST150).

In the user terminal 11, the screen data of the prediction evaluation result display screen 95 is reproduced under the control of the browser control unit 82, and the reproduced prediction evaluation result display screen 95 is displayed on the display 24B. As a result, the prediction information 15 is presented to the user U.

As described above, the information processing server 10 uses the first prediction model 311 and the second prediction model 312 as the prediction model 31 for predicting the evaluation result of the Ames mutagenicity test for the candidate substance. The first prediction model 311 outputs the first prediction evaluation result 601 indicating whether or not the candidate substance has mutagenicity related to the base pair substitution mutation in a case where the candidate substance is added to the first strain having sensitivity to the base pair substitution mutation in which a part of a base sequence is changed. The second prediction model 312 outputs the second prediction evaluation result 602 indicating whether or not the candidate substance has mutagenicity related to the frameshift mutation in which a reading frame of a base sequence is shifted, in a case where the candidate substance is added to the second strain having sensitivity to the frameshift mutation.

The CPU 22A of the information processing server 10 functions as the request reception unit 35, the prediction unit 38, and the screen delivery control unit 39. The request reception unit 35 acquires the candidate substance information 14 included in the prediction request 13 by receiving the prediction request 13 from the user terminal 11. The prediction unit 38 inputs the candidate substance information 14 to the first prediction model 311 and inputs the feature amount information 42 derived from the candidate substance information 14 to the second prediction model 312. Then, the first prediction evaluation result 601 and the second prediction evaluation result 602 are output from the first prediction model 311 and the second prediction model 312, respectively. The screen delivery control unit 39 delivers the screen data of the prediction evaluation result display screen 95 including the prediction information 15 corresponding to the first prediction evaluation result 601 and the second prediction evaluation result 602 to the user terminal 11 to present the prediction information 15 to the user U. Therefore, it is possible to improve the prediction accuracy of the evaluation result of the Ames mutagenicity test as compared with a case in which the prediction model common to both the base pair substitution mutation and the frameshift mutation, which have completely different causes of occurrence, structural features, and the like, is used. As a result, it is possible to improve the identification accuracy of whether the type of the mutation of the gene is the base pair substitution mutation or the frameshift mutation.

As shown in FIGS. 10, 11, and 14, in a case where both the first prediction evaluation result 601 and the second prediction evaluation result 602 indicate that the candidate substance does not have mutagenicity, the screen delivery control unit 39 presents to the user U a fact that the candidate substance does not have mutagenicity as the prediction information 15. On the other hand, in a case where at least one of the first prediction evaluation result 601 or the second prediction evaluation result 602 indicates that the candidate substance has mutagenicity, the screen delivery control unit 39 presents to the user U a fact that the candidate substance has mutagenicity as the prediction information 15. Therefore, the user U can correctly understand the prediction of the evaluation result of the Ames mutagenicity test for determining whether or not the candidate substance has mutagenicity.

In addition, the screen delivery control unit 39 presents the first prediction evaluation result 601 and the second prediction evaluation result 602 themselves to the user U as the prediction information 15. Therefore, the user U can understand whether or not the candidate substance has mutagenicity related to the base pair substitution mutation and whether or not the candidate substance has mutagenicity related to the frameshift mutation. The user U can use, for example, the comparison between the chemical structure of the candidate substance having the mutagenicity related to the base pair substitution mutation and the chemical structure of the candidate substance not having the mutagenicity related to the base pair substitution mutation as a guide for the subsequent design of the candidate substance, and the like.

As shown in FIG. 1, the candidate substance information 14 is information related to the chemical structure of the candidate substance. The information related to the chemical structure is information that most directly represents the properties of the candidate substance. Therefore, the first prediction evaluation result 601 reflecting the properties of the candidate substance can be output from the first prediction model 311.

As shown in FIGS. 5 and 6, the prediction model 31 is constructed by any machine learning method of a support vector machine, linear separation, a gradient boosting tree, AdaBoost, a random forest, deep learning, or ensemble learning thereof. Since these are all very general machine learning methods, it is possible to easily construct a prediction model 31 having relatively high prediction accuracy.

In addition, the first prediction model 311 and the second prediction model 312 are constructed by different machine learning methods. Therefore, the first prediction model 311 suitable for predicting whether or not the candidate substance has the mutagenicity related to the base pair substitution mutation and the second prediction model 312 suitable for predicting whether or not the candidate substance has the mutagenicity related to the frameshift mutation can be constructed.

Furthermore, the first prediction model 311 is constructed by deep learning, and the second prediction model 312 is constructed by a machine learning method other than deep learning.

The base pair substitution mutation is a main cause of mutagenicity, and there is more data that can be referred to than the frameshift mutation that is a secondary cause of mutagenicity. That is, the first training data 621 is larger than the second training data 622. Therefore, deep learning that requires a large amount of training data to improve the prediction accuracy is suitable for the first prediction model 311. On the other hand, for the second prediction model 312, a machine learning method other than deep learning that can expect improvement in prediction accuracy by using selected feature amounts even with a relatively small amount of training data is suitable. As described above, by constructing the first prediction model 311 and the second prediction model 312 by machine learning methods in which each of the first prediction model 311 and the second prediction model 312 is skilled, it is possible to further improve the prediction accuracy of the evaluation result of the Ames mutagenicity test. It should be noted that a plurality of prediction models 31 may be constructed by a plurality of different machine learning methods, and the prediction model 31 having the highest prediction accuracy among the plurality of prediction models 31 may be adopted.

The CPU 22A of the information processing server 10 further functions as the feature amount derivation unit 37. The feature amount derivation unit 37 derives the feature amount information 42 from the candidate substance information 14 to acquire the feature amount information 42. The prediction unit 38 inputs the feature amount information 42 to the second prediction model 312 constructed by the support vector machine. Therefore, the second prediction evaluation result 602 can be smoothly output from the second prediction model 312.

As shown in FIG. 4, the feature amount information 42 includes the feature amount 45 related to the geometric shape of the candidate substance, the feature amount 46 related to the electronic physical property of the candidate substance, the feature amount 47 related to the physicochemical property of the candidate substance, and the feature amount 48 related to the partial structure of the candidate substance. Therefore, the prediction accuracy of the second prediction evaluation result 602 can be improved. The feature amount information 42 may include at least one of the feature amounts 45 to 48.

As shown in FIG. 9, the first training data 621 used for training the first prediction model 311 and the second training data 622 used for training the second prediction model 312 are at least partially different from each other. Therefore, it is possible to perform training corresponding to each of the first prediction model 311 and the second prediction model 312.

In addition, as shown in FIG. 9, the first training data 621 and the second training data 622 are prepared based on the information on the first strain and the second strain. Therefore, the first training data 621 and the second training data 622 suitable for each of the first prediction model 311 and the second prediction model 312 can be prepared.

### (Modification Example 1)

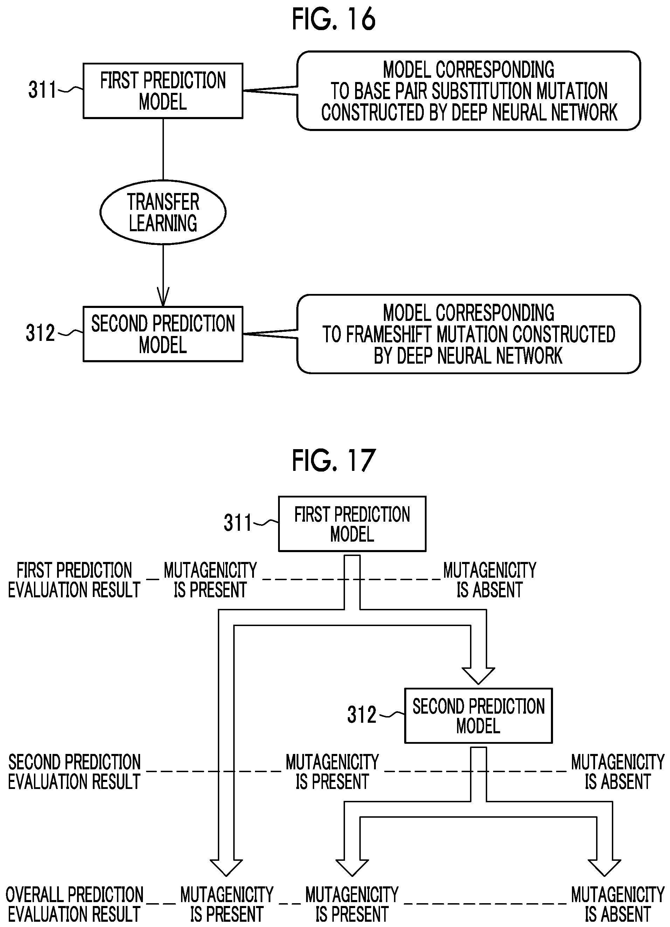

As shown in FIG. 16 as an example, the second prediction model 312 corresponding to the frameshift mutation may be constructed by performing transfer learning on the first prediction model 311 corresponding to the base pair substitution mutation, which is constructed by the deep neural network. In the transfer learning, as shown in FIG. 7, the same processing as that shown in FIG. 7 is performed on the trained first prediction model 311 trained by the first training data 621 by the second training data 622. The first prediction model 311 is trained by the first training data 621 having a larger number than the second training data 622, and is used as the second prediction model 312 by the transfer learning. Therefore, it is possible to suppress a decrease in the prediction accuracy of the second prediction model 312 due to the small number of pieces of the second training data 622 as compared with a case in which the first prediction model 311 and the second prediction model 312 constructed by the deep neural network are separately prepared. That is, with this configuration as well, it is possible to solve the problem in the related art that the prediction accuracy of the prediction model 31 cannot be improved because the number of past Ames mutagenicity test data that can be used as the training data is small. In addition, in this case, since it is not necessary to derive the feature amount information 42, the processing can be simplified. It should be noted that, in this case, the internal parameters such as the coefficient of the filter of the first prediction model 311 and the second prediction model 312 are different.

### (Modification Example 2)

In the above example, the prediction of whether or not the candidate substance has the mutagenicity related to the base pair substitution mutation using the first prediction model 311 and the prediction of whether or not the candidate substance has the mutagenicity related to the frameshift mutation using the second prediction model 312 are performed in parallel in the prediction unit 38, but the present disclosure is not limited to this. The prediction may be performed by a procedure shown in FIG. 17 as an example. That is, first, whether or not the candidate substance has the mutagenicity related to the base pair substitution mutation is predicted using the first prediction model 311. Then, in a case where the first prediction evaluation result 601 indicates that the candidate substance does not have the mutagenicity related to the base pair substitution mutation, whether or not the candidate substance has the mutagenicity related to the frameshift mutation is predicted using the second prediction model 312.

In this case as well, as in the above example, in a case where both the first prediction evaluation result 601 and the second prediction evaluation result 602 indicate that the candidate substance does not have mutagenicity, the prediction unit 38 outputs the overall prediction evaluation result 76 indicating that the candidate substance does not have mutagenicity. On the other hand, in a case where at least one of the first prediction evaluation result 601 or the second prediction evaluation result 602 indicates that the candidate substance has mutagenicity, the prediction unit 38 outputs the overall prediction evaluation result 76 indicating that the candidate substance has mutagenicity. In a case where the first prediction evaluation result 601 indicates that the candidate substance has mutagenicity, the prediction using the second prediction model 312 is not performed, so that the processing time can be shortened. However, as in the above example, it is preferable to always perform the prediction using the first prediction model 311 and the prediction using the second prediction model 312 because the reliability of the overall prediction evaluation result 76 is increased.

### (Modification Example 3)

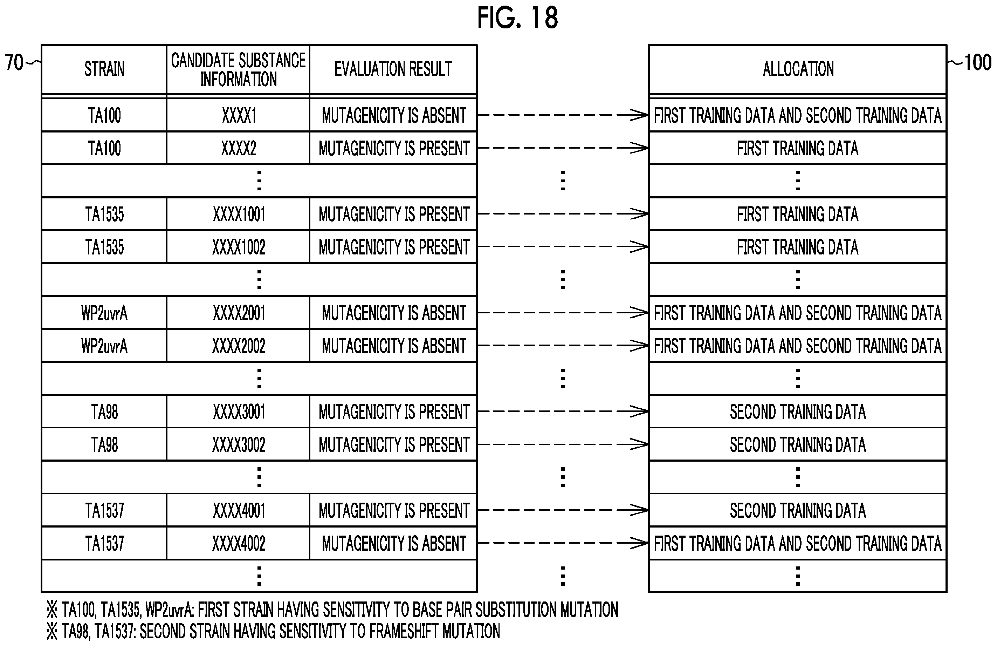

The allocation method of the first training data 621 and the second training data 622 is not limited to the example shown in FIG. 9. As shown in Table 100 in FIG. 18 as an example, among the Ames mutagenicity tests that have been actually performed in the past, those registered as having mutagenicity in the evaluation result are allocated based on the information on the strain as in the case of FIG. 9, but those registered as not having mutagenicity in the evaluation result may be allocated to both the first training data 621 and the second training data 622. That is, the first training data 621 and the second training data 622 may be at least partially different from each other.

### (Modification Example 4)

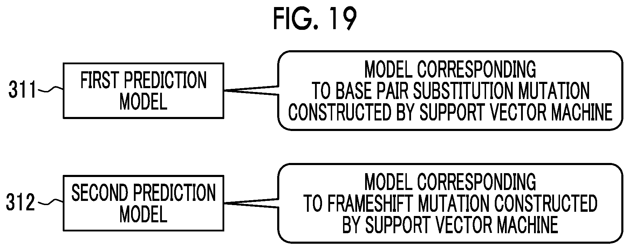

In FIGS. 5 and 6, an example is shown in which the first prediction model 311 and the second prediction model 312 are constructed by different machine learning methods (the first prediction model 311 is constructed by the deep neural network, and the second prediction model 312 is constructed by the support vector machine), but the present disclosure is not limited to this. As shown in FIG. 19 as an example, the first prediction model 311 and the second prediction model 312 may be constructed by the same machine learning method. FIG. 19 shows a case in which both the first prediction model 311 and the second prediction model 312 are constructed by the support vector machine.

However, in the case of FIG. 19, even though the machine learning method is the same support vector machine, as shown in FIGS. 20 and 21 as an example, it is obvious that the first boundary line 661 in the feature amount space 65 of the first prediction model 311 and the second boundary line 662 in the feature amount space 65 of the second prediction model 312 are different from each other. The first boundary line 661 and the second boundary line 662 are examples of an "internal parameter" according to the technology of the present disclosure. Even in a case where the first prediction model 311 and the second prediction model 312 are constructed by the same machine learning method, the first prediction model 311 suitable for predicting whether or not the candidate substance has the mutagenicity related to the base pair substitution mutation and the second prediction model 312 suitable for predicting whether or not the candidate substance has the mutagenicity related to the frameshift mutation can be used. In this case, as shown in FIG. 20, the first training data 621 is composed of a set of training feature amount information 42L and first correct answer data 601CA.

Both the first prediction model 311 and the second prediction model 312 may be constructed by deep learning such as a deep neural network. The internal parameter in this case is, for example, a coefficient set in an intermediate layer (hidden layer) or a coefficient of a filter used for a convolution operation.

The candidate substance information 14 is not limited to a character string representing the chemical structure of the candidate substance by the exemplary SMILES notation. A molecular design limited (MDL) file representing the chemical structure of the candidate substance, a structure-data file (SDF), or the like may be used. In any case, a description method that can uniquely determine a three-dimensional structure such as an isomer is preferable, and a description method that can represent three-dimensional coordinate information of a molecule is more preferable.

The feature amount information 42 may be input to the information processing server 10 in a form of being derived by a device different from the information processing server 10. In addition, the user U may input the feature amount information 42 via the input device 25B of the user terminal 11.

The prediction information 15 may be only the overall prediction evaluation result 76. On the contrary, the prediction information 15 may be only a set of the first prediction evaluation result 601 and the second prediction evaluation result 602.

The information processing server 10 may be installed in the pharmaceutical company or the CRO, or may be installed in a data center independent of the pharmaceutical company or the CRO.

The prediction information 15 itself may be delivered to the user terminal 11 instead of delivering the screen data of the prediction evaluation result display screen 95 including the prediction information 15 to the user terminal 11. In this case, in the user terminal 11, the prediction evaluation result display screen 95 is generated based on the prediction information 15 under the control of the browser control unit 82.

The method of presenting the prediction information 15 to the user U is not limited to the presentation by the delivery of the exemplary screen data. The prediction information 15 may be presented to the user U by printing the prediction information 15 on a paper medium, or may be presented by transmitting the prediction information 15 attached to an electronic mail to the user terminal 11.

The product is not limited to a drug. The product may be a cosmetic or the like.

The hardware configuration of the computer constituting the information processing server 10 according to the technology of the present disclosure can be variously modified. For example, the information processing server 10 can be configured by using a plurality of computers separated as hardware for the purpose of improving processing ability and reliability. For example, functions of the request reception unit 35 and the RW control unit 36 and the feature amount derivation unit 37, the prediction unit 38, and the screen delivery control unit 39 are provided in a distributed manner between two computers. In this case, the information processing server 10 is configured by using two computers. The user terminal 11 may perform a part or all of the functions of the information processing server 10.

As described above, the hardware configuration of the computer of the information processing server 10 can be changed as appropriate in accordance with required performance, such as processing capacity, safety, and reliability. Not only the hardware but also the APs such as the operation program 30 may be duplicated or stored in a distributed manner between a plurality of storages for the purpose of securing safety and reliability.

In the above-described embodiment, for example, a hardware structure of a processing unit that executes various types of processing, such as the request reception unit 35, the RW control unit 36, the feature amount derivation unit 37, the prediction unit 38, the screen delivery control unit 39, and the browser control unit 82, can use the following various processors. The various processors include, for example, the CPUs 22A and 22B which are general-purpose processors executing software (the operation program 30 and the prediction AP 80) to function as various processing units as described above, a programmable logic device (PLD), such as a field programmable gate array (FPGA), which is a processor whose circuit configuration can be changed after manufacture, and a dedicated electric circuit, such as an application specific integrated circuit (ASIC), which is a processor having a dedicated circuit configuration designed to perform a specific process.

One processing unit may be configured by one of these various processors or by a combination of two or more processors of the same type or different types (for example, a combination of a plurality of FPGAs and/or a combination of a CPU and an FPGA). Further, a plurality of processing units may be configured with one processor.

As an example in which a plurality of processing units are configured using a single processor, first, there is a form in which a processor is configured using a combination of at least one CPU and software, as represented by a computer such as a client or a server, and the processor functions as a plurality of processing units. Second, as represented by a system on a chip (SoC) or the like, there is a form in which a processor, which implements the functions of the entire system including the plurality of processing units with a single integrated circuit (IC) chip, is used. As described above, the various types of processing units are configured using one or more of the above-described various types of processors as a hardware structure.

Furthermore, specifically, an electric circuit (circuitry) obtained by combining circuit elements, such as semiconductor elements, can be used as the hardware structure of the various processors.

It is possible to understand the technology according to the following supplementary notes, based on the above description.

### [Supplementary Note 1]

An information processing apparatus that predicts an evaluation result of an Ames mutagenicity test for a candidate substance for a product using a prediction model, the information processing apparatus comprising: a processor,
wherein the processor is configured to:
use, as the prediction model,
   a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and
   a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation;
acquire candidate substance information related to the candidate substance;
input the candidate substance information or information based on the candidate substance information to the first prediction model and the second prediction model, and output the first prediction evaluation result and the second prediction evaluation result from the first prediction model and the second prediction model; and
present prediction information corresponding to the first prediction evaluation result and the second prediction evaluation result to a user.

### [Supplementary Note 2]

The information processing apparatus according to supplementary note 1,
wherein the processor is configured to:
present, in a case where both the first prediction evaluation result and the second prediction evaluation result indicate that the candidate substance does not have mutagenicity, a fact that the candidate substance does not have mutagenicity to the user as the prediction information; and
present, in a case where at least one of the first prediction evaluation result or the second prediction evaluation result indicates that the candidate substance has mutagenicity, a fact that the candidate substance has mutagenicity to the user as the prediction information.

### [Supplementary Note 3]

The information processing apparatus according to supplementary note 1 or 2,
wherein the processor is configured to present the first prediction evaluation result and the second prediction evaluation result themselves to the user as the prediction information.

### [Supplementary Note 4]

The information processing apparatus according to any one of supplementary notes 1 to 3,
wherein the candidate substance information is information related to a chemical structure of the candidate substance.

### [Supplementary Note 5]

The information processing apparatus according to any one of supplementary notes 1 to 4,
wherein the prediction model is constructed by any machine learning method of a support vector machine, linear separation, a gradient boosting tree, AdaBoost, a random forest, deep learning, or ensemble learning thereof.

### [Supplementary Note 6]

The information processing apparatus according to supplementary note 5,
wherein the first prediction model is constructed by deep learning, and
the second prediction model is constructed by performing transfer learning on the trained first prediction model.

### [Supplementary Note 7]

The information processing apparatus according to supplementary note 5,
wherein the first prediction model and the second prediction model are constructed by different machine learning methods.

### [Supplementary Note 8]

The information processing apparatus according to supplementary note 7,
wherein the first prediction model is constructed by deep learning, and
the second prediction model is constructed by a machine learning method other than deep learning.

### [Supplementary Note 9]

The information processing apparatus according to supplementary note 5 or 6,
wherein the first prediction model and the second prediction model are constructed by the same machine learning method while internal parameters for deriving the first prediction evaluation result and the second prediction evaluation result are different.

### [Supplementary Note 10]

The information processing apparatus according to any one of supplementary notes 5, 7, 8, or 9,
wherein in a case where the prediction model is constructed by any machine learning method of a support vector machine, linear separation, a gradient boosting tree, AdaBoost, a random forest, or ensemble learning thereof,
the processor is configured to:
   acquire feature amount information of the candidate substance; and
   input the feature amount information to the prediction model.

### [Supplementary Note 11]

The information processing apparatus according to supplementary note 10,
wherein the feature amount information includes at least one of a feature amount related to a geometric shape of the candidate substance, a feature amount related to an electronic physical property of the candidate substance, a feature amount related to a physicochemical property of the candidate substance, or a feature amount related to a partial structure of the candidate substance.

### [Supplementary Note 12]

The information processing apparatus according to any one of supplementary notes 1 to 11,
wherein first training data used for training the first prediction model and second training data used for training the second prediction model are at least partially different from each other.

### [Supplementary Note 13]

The information processing apparatus according to any one of supplementary notes 1 to 12,
wherein first training data used for training the first prediction model and second training data used for training the second prediction model are prepared based on information on the first strain and the second strain.

The technology of the present disclosure can also be combined with various embodiments and/or various modification examples described above, as appropriate. The disclosed technology is not limited to the above embodiment and may adopt various configurations without departing from its gist. Furthermore, the technology of the present disclosure extends to a storage medium that non-transitorily stores the program, and a computer program product including the program, in addition to the program.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In order to avoid complications and facilitate grasping the parts according to the technology of the present disclosure, in the above-described contents and the above-shown contents, the description of technical general knowledge and the like that do not particularly require description for enabling the implementation of the technology of the present disclosure is omitted.

In the present specification, "A and/or B" is synonymous with "at least one of A or B". That is, "A and/or B" means that it may be only A, only B, or a combination of A and B. Further, in the present specification, in a case where three or more items are expressed in combination using "and/or", the same concept as that of "A and/or B" applies.

All of the publications, the patent applications, and the technical standards described in the specification are incorporated by reference herein to the same extent as each individual document, each patent application, and each technical standard are specifically and individually stated to be incorporated by reference.

## Claims

1. An information processing apparatus that predicts an evaluation result of an Ames mutagenicity test for a candidate substance for a product using a prediction model, the information processing apparatus comprising: a processor,
wherein the processor is configured to:
use, as the prediction model,
a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and
a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation;
acquire candidate substance information related to the candidate substance;
input the candidate substance information or information based on the candidate substance information to the first prediction model and the second prediction model, and output the first prediction evaluation result and the second prediction evaluation result from the first prediction model and the second prediction model; and
present prediction information corresponding to the first prediction evaluation result and the second prediction evaluation result to a user.

2. The information processing apparatus according to claim 1,
wherein the processor is configured to:
present, in a case where both the first prediction evaluation result and the second prediction evaluation result indicate that the candidate substance does not have mutagenicity, a fact that the candidate substance does not have mutagenicity to the user as the prediction information; and
present, in a case where at least one of the first prediction evaluation result or the second prediction evaluation result indicates that the candidate substance has mutagenicity, a fact that the candidate substance has mutagenicity to the user as the prediction information.

3. The information processing apparatus according to claim 1,
wherein the processor is configured to present the first prediction evaluation result and the second prediction evaluation result themselves to the user as the prediction information.

4. The information processing apparatus according to claim 1,
wherein the candidate substance information is information related to a chemical structure of the candidate substance.

5. The information processing apparatus according to claim 1,
wherein the prediction model is constructed by any machine learning method of a support vector machine, linear separation, a gradient boosting tree, AdaBoost, a random forest, deep learning, or ensemble learning thereof.

6. The information processing apparatus according to claim 5,
wherein the first prediction model is constructed by deep learning, and
the second prediction model is constructed by performing transfer learning on the trained first prediction model.

7. The information processing apparatus according to claim 5,
wherein the first prediction model and the second prediction model are constructed by different machine learning methods.

8. The information processing apparatus according to claim 7,
wherein the first prediction model is constructed by deep learning, and
the second prediction model is constructed by a machine learning method other than deep learning.

9. The information processing apparatus according to claim 5,
wherein the first prediction model and the second prediction model are constructed by the same machine learning method while internal parameters for deriving the first prediction evaluation result and the second prediction evaluation result are different.

10. The information processing apparatus according to claim 5,
wherein in a case where the prediction model is constructed by any machine learning method of a support vector machine, linear separation, a gradient boosting tree, AdaBoost, a random forest, or ensemble learning thereof,
the processor is configured to:
acquire feature amount information of the candidate substance; and
input the feature amount information to the prediction model.

11. The information processing apparatus according to claim 10,
wherein the feature amount information includes at least one of a feature amount related to a geometric shape of the candidate substance, a feature amount related to an electronic physical property of the candidate substance, a feature amount related to a physicochemical property of the candidate substance, or a feature amount related to a partial structure of the candidate substance.

12. The information processing apparatus according to claim 1,
wherein first training data used for training the first prediction model and second training data used for training the second prediction model are at least partially different from each other.

13. The information processing apparatus according to claim 1,
wherein first training data used for training the first prediction model and second training data used for training the second prediction model are prepared based on information on the first strain and the second strain.

14. An operation method of an information processing apparatus that predicts an evaluation result of an Ames mutagenicity test for a candidate substance for a product using a prediction model, the operation method comprising:
using, as the prediction model,
a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and
a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation;
acquiring candidate substance information related to the candidate substance;
inputting the candidate substance information or information based on the candidate substance information to the first prediction model and the second prediction model, and outputting the first prediction evaluation result and the second prediction evaluation result from the first prediction model and the second prediction model; and
presenting prediction information corresponding to the first prediction evaluation result and the second prediction evaluation result to a user.

15. An operation program of an information processing apparatus that predicts an evaluation result of an Ames mutagenicity test for a candidate substance for a product using a prediction model, the operation program causing a computer to execute a process comprising:
using, as the prediction model,
a first prediction model that outputs, in a case where the candidate substance is added to a first strain having sensitivity to a base pair substitution mutation in which a part of a base sequence is changed, a first prediction evaluation result indicating whether or not the candidate substance has mutagenicity related to the base pair substitution mutation, and
a second prediction model that outputs, in a case where the candidate substance is added to a second strain having sensitivity to a frameshift mutation in which a reading frame of a base sequence is shifted, a second prediction evaluation result indicating whether or not the candidate substance has mutagenicity related to the frameshift mutation;
acquiring candidate substance information related to the candidate substance;
inputting the candidate substance information or information based on the candidate substance information to the first prediction model and the second prediction model, and outputting the first prediction evaluation result and the second prediction evaluation result from the first prediction model and the second prediction model; and
presenting prediction information corresponding to the first prediction evaluation result and the second prediction evaluation result to a user.
